(19) Europäisches Patentamt
European Patent Office
Office européen des brevets

(11) EP 4 744 692 A1

(12) **EUROPEAN PATENT APPLICATION**
published in accordance with Art. 153(4) EPC

(43) Date of publication:
**20.05.2026 Bulletin 2026/21**

(21) Application number: 25758156.1

(22) Date of filing: **22.01.2025**

(51) International Patent Classification (IPC):
**A61M 1/34** (2006.01)    **A61M 1/16** (2006.01)

(52) Cooperative Patent Classification (CPC):
**A61M 1/16; A61M 1/34**

(86) International application number:
**PCT/JP2025/001903**

(87) International publication number:
**WO 2025/177756 (28.08.2025 Gazette 2025/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.02.2024 JP 2024025785**

(71) Applicant: Physiologas Technologies, Inc.
**Sagamihara-shi, Kanagawa 252-0373 (JP)**

(72) Inventors:
• **MIYAWAKI, Kazuyoshi**
**Sagamihara-shi, Kanagawa 252-0373 (JP)**
• **KOKUBO, Kenichi**
**Sagamihara-shi, Kanagawa 252-0373 (JP)**
• **AOKI, Hiroyuki**
**Sagamihara-shi, Kanagawa 252-0373 (JP)**

(74) Representative: **Schön, Christoph**
**Dr. Schön, Neymeyr & Partner mbB**
**Bavariaring 26**
**80336 München (DE)**

(54) **TARGET LIQUID PURIFICATION DEVICE**

(57)    An object of the present invention is to provide a target liquid purification device capable of reliably performing dialysis and accurately measuring a concentration of a target substance in blood while reducing a total volume of a dialysate used in the dialysis despite having a structure which is simple and safe enough to have no adverse effect on the human body and which does not require drawing blood. The target liquid purification device according to the present invention includes a main body portion 1, a concentration measurement device 2, a control device 3, and a notification device 4. The main body portion 1 includes a separation device 100, an unpurified target liquid passage 11, a purified target liquid passage 12, a filtration dialysate passage 21, a dialysate passage 412, an effluent passage 24, a first flow rate adjustment device 36, and a concentration sensor 35 that outputs a signal according to a concentration of a target substance in a target liquid. The concentration measurement device 2 measures a concentration of target substances in a target liquid based on an output signal of the concentration sensor 35 in a time period where the first flow rate adjustment device 36 controls a flow rate of a filtration dialysate to or below a flow rate of an unpurified target liquid.

Fig. 1:

FIG.1

**Description**

Technical Field

The present invention relates to a device for purifying a target liquid.

Background Art

Conventionally, blood purification methods involving partial regeneration using adsorbents have been proposed with the aim of reducing a total volume of dialysate used in dialysis methods (for example, refer to Patent Literature 1).

Citation List

Patent Literature

Patent Literature 1: U.S. Patent No. 9302038

Summary of Invention

Technical Problem

However, according to the dialysate used in the dialysis method disclosed in Patent Literature 1, there is a problem in that the total amount of dialysate remains high because a replenishment fluid is supplied during dialysis. There is also a problem in that a large amount of the replenishment liquid is necessary. In other words, the dialysis method disclosed in Patent Literature 1 is an insufficient method from the perspective of reducing the total amount of dialysate.

In addition, generally, there is a problem in that reducing the total volume of dialysate prevents dialysis from being sufficiently performed. From the perspective of reducing the total amount of dialysate, conceivable methods of determining whether dialysis has been sufficiently performed include measuring a concentration of a target substance in a target liquid (blood) during target liquid purification (during dialysis) and implanting a measurement device for measuring the target substance directly inside the human body. However, when implanting the measurement device into the human body, from a safety perspective, the device or dialysis method becomes complex, creating problems in terms of both safety and efficiency.

Other conceivable methods include drawing blood during dialysis without providing a measurement device. However, since this method requires separately drawing blood through a fluid passage other than a fluid passage connected to a dialyzer during the dialysis, from a safety perspective, the device or dialysis method becomes complex, creating problems in terms of both safety and efficiency.

Yet other conceivable methods include providing a measurement device inside a dialyzer. However, with this method, since blood only comes into contact with a porous membrane for a short period of time during dialysis, a difference in concentration arises between before and after passing through the porous membrane, creating a problem in that the concentration of a target substance in blood cannot be accurately measured.

In consideration thereof, an object of the present invention is to provide a target liquid purification device capable of reliably performing dialysis and accurately measuring a concentration of a target substance in blood while reducing a total volume of a dialysate used in the dialysis with a structure which is simple and safe enough to have no adverse effect on the human body and which does not require drawing blood.

Solution to Problem

In order to achieve the object described above, the target liquid purification device includes the following matters that specify the invention.

The target liquid purification device is
a target liquid purification device that removes at least one of a plurality of target substances from an unpurified target liquid, the target liquid purification device comprising:

a separation device including a porous membrane having a pore size through which the target substances contained in the unpurified target liquid can pass and a first portion and a second portion separated by the porous membrane, the separation device being configured to generate a filtration dialysate by introducing the unpurified target liquid containing the target substances from the first portion into the second portion while passing the unpurified target liquid through the porous membrane and to generate a purified target liquid by removing the target substances contained in the unpurified target liquid by the pass-through;

an unpurified target liquid passage configured to introduce the unpurified target liquid into the first portion of the separation device;

a purified target liquid passage configured to guide the purified target liquid out from the first portion of the separation device;

a filtration dialysate passage configured to guide the filtration dialysate out from a guiding portion of the second portion of the separation device;

an effluent passage configured to discard the filtration dialysate from the filtration dialysate passage as an effluent;

a dialysate passage configured to introduce a dialysate into an introducing portion provided upstream of the guiding portion of the second portion;

a concentration sensor which is provided in at least one of the filtration dialysate passage and the effluent passage and which is configured to output a signal according to the concentration of the target substances in the filtration dialysate;

a flow rate adjustment device which is provided in at least one of the filtration dialysate passage and the effluent passage and which is configured to adjust a flow rate of the filtration dialysate; and

a concentration measurement device configured to measure the concentration of the target substances based on the output signal of the concentration sensor in a time period where the flow rate adjustment device controls the flow rate of the filtration dialysate to or below a flow rate of the unpurified target liquid.

Preferably, the target liquid purification device with the configuration described above further comprises:

a replenishment liquid passage configured to introduce a replenishment liquid into the target liquid purification device, wherein

the replenishment liquid passage is configured to introduce the replenishment liquid into at least one of the unpurified target liquid passage, the purified target liquid passage, and the dialysate passage.

Preferably, in the target liquid purification device with any of the configurations described above,

the effluent passage branches from the filtration dialysate passage and is configured to discard a first liquid being a part of the filtration dialysate as an effluent; and

the filtration dialysate passage is configured to introduce a second liquid being a remainder of the filtration dialysate as a dialysate into an introducing portion of the dialysate passage.

Preferably, the target liquid purification device with any of the configurations described above further comprises:

a replenishment liquid passage configured to introduce a replenishment liquid into the target liquid purification device, wherein

the replenishment liquid passage is configured to introduce the replenishment liquid into at least one of the unpurified target liquid passage, the purified target liquid passage, the filtration dialysate passage, and the dialysate passage.

Preferably, the target liquid purification device with any of the configurations described above comprises:

a regenerating unit which is provided between a branching point of the effluent passage and the introducing portion of the dialysate passage in the filtration dialysate passage, which includes an adsorbent configured to adsorb the target substances, and which is configured to remove at least one of the target substances from the second liquid by bringing the second liquid having been introduced from the filtration dialysate passage in contact with the adsorbent and to generate a regenerated liquid with a reduced concentration of the target substances, wherein

the regenerating unit is configured to introduce the regenerated liquid as a dialysate into the introducing portion of the dialysate passage.

Preferably, the target liquid purification device with any of the configurations described above further comprises:

a replenishment liquid passage configured to introduce a replenishment liquid into the target liquid purification device, wherein

the replenishment liquid passage is configured to introduce the replenishment liquid into at least one of the unpurified target liquid passage, the purified target liquid passage, the filtration dialysate passage, and the dialysate passage.

Preferably, the target liquid purification device with any of the configurations described above comprises:
a effluent liquid flow rate adjustment device configured to adjust a flow rate of the first liquid and a replenishment liquid flow rate adjustment device configured to adjust a flow rate of the replenishment liquid.

Preferably, the target liquid purification device with any of the configurations described above comprises:

an abnormality detection device configured to detect an abnormality based on a time-series variation in the concentration of the target substances; and
a notification device configured to notify an abnormality detected by the abnormality detection device.

Advantageous Effects of Invention

According to the target liquid purification device with any of the configurations described above, the concentration sensor and the flow rate adjustment device are provided in at least one of the filtration dialysate passage and the effluent passage. Therefore, a degree of purification in a target liquid can be estimated without having to separately draw blood.

In addition, the concentration measurement device is configured to measure a concentration of target substances based on an output signal of the concentration sensor in a time period where the flow rate adjustment device controls the flow rate of a filtration dialysate to or below a flow rate of an unpurified target liquid. Therefore, when the concentration measurement device measures the concentration of target substances in a target liquid (such as blood), the unpurified target liquid is to come into prolonged contact with a porous membrane. As a result, the concentration of the target substances in the unpurified target liquid and the concentration of the target substances in the filtration dialysate become approximately the same. Therefore, the concentration of the target substance in the unpurified target liquid can be accurately, efficiently, and safely measured without having to separately draw blood or insert the concentration measurement device into the human body.

Brief Description of Drawings

FIG. 1 is an explanatory configuration diagram of a target liquid purification device.
FIG. 2 is an explanatory configuration diagram of a main body portion of a target liquid purification device as a first embodiment.
FIG. 3 is an explanatory configuration diagram of a main body portion of a target liquid purification device as a second embodiment.
FIG. 4 is an explanatory configuration diagram of a main body portion of a target liquid purification device as a third embodiment.
FIG. 5 is an explanatory configuration diagram of a main body portion of a target liquid purification device as a fourth embodiment.
FIG. 6 is a diagram showing a concentration of urea nitrogen in an unpurified target liquid and a concentration of urea nitrogen in a first liquid for each flow rate of a replenishment liquid in a target liquid purification device according to an example.
FIG. 7 is a diagram showing a relationship between $Q_D$ (replenishment liquid flow rate) and $C_{DO}$ (urea nitrogen concentration in first liquid) in the target liquid purification device according to the example.
FIG. 8A is a diagram showing a relationship between a urea nitrogen concentration and a position of a separation device as viewed from an unpurified target liquid passage when the flow rate of the replenishment liquid is set to 500 mL/min in the target liquid purification device according to the example.
FIG. 8B is a diagram showing a relationship between a urea nitrogen concentration and a position of the separation device as viewed from an unpurified target liquid passage when the flow rate of the replenishment liquid is set to 230 mL/min in the target liquid purification device according to the example.
FIG. 8C is a diagram showing a relationship between a urea nitrogen concentration and a position of the separation device as viewed from an unpurified target liquid passage when the flow rate of the replenishment liquid is set to 150 mL/min in the target liquid purification device according to the example.

Description of Embodiments

(First embodiment)

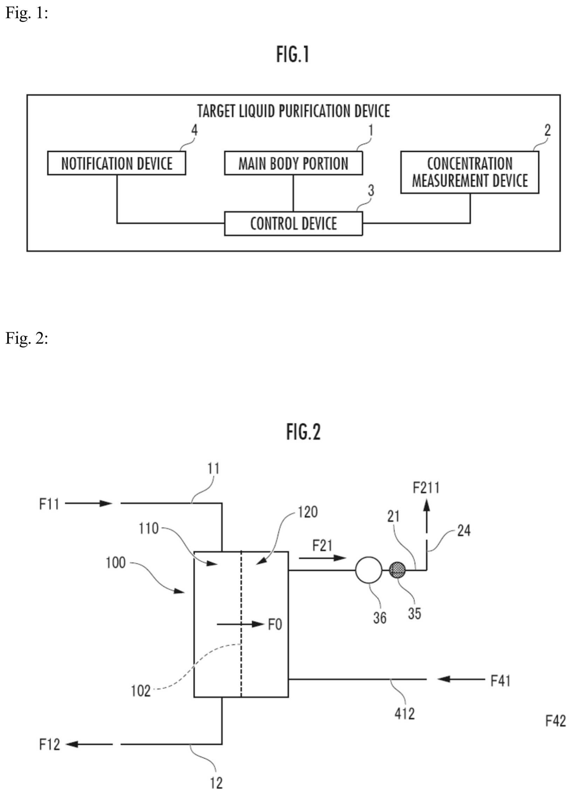

A target liquid purification device as a first embodiment shown in FIG. 1 comprises a main body portion 1, a concentration measurement device 2, a control device 3, and a notification device 4. FIG. 2 shows a schematic diagram related to the first embodiment of the main body portion 1 in FIG. 1. As shown in FIG. 2, the main body portion 1 comprises a separation device 100, an unpurified target liquid passage 11, a purified target liquid passage 12, a filtration dialysate passage 21, a dialysate passage 412, an effluent passage 24, a first flow rate adjustment device 36, and a concentration sensor 35 that outputs a signal according to a concentration of a target substance in a target liquid. The concentration measurement device 2 measures a concentration of the target substance in the target liquid based on an output signal of the concentration sensor 35.

The separation device 100 is divided into a first portion 110 and a second portion 120 by a porous membrane 102. The porous membrane 102 has a pore size that allows a target substance contained in an unpurified target liquid F11 (for example, blood, plasma, a dialysis effluent, or a blood filtrate) to pass through. The "target substance" is, for example, at least one ion among potassium ions, ammonium ions, calcium ions, magnesium ions, phosphate ions, hydrogen carbonate ions, and organic acid ions or a disease agent that accumulates in the body when one becomes ill such as urea, creatinine, uric acid, peptides, and proteins. The separation device 100 generates a filtration dialysate F21 by introducing the unpurified target liquid F11 containing the target substance from the first portion 110 into the second portion 120 by passing the unpurified target liquid F11 through the porous membrane 102. The separation device 100 is configured to generate a purified target liquid F12 by removing the target substance contained in the unpurified target liquid F11 by the pass-through. Note that a fluid that moves from the first portion 110 to the second portion 120 at this point is an ultrafiltration liquid F0.

In this case, the "ultrafiltration liquid" refers to excess water contained in the body. The water is preferably discharged during purification of the target liquid. The amount of the ultrafiltration liquid F0 typically varies from person to person and is calculated using a predetermined calculation method based on factors such as the user's weight, meal frequency, or height. For a typical person (for example, a person weighing 60 kg or less), a flow rate of the ultrafiltration liquid F0 is preferably kept within 1000 mL per hour.

The unpurified target liquid passage 11 is configured to introduce the unpurified target liquid F11 guided out from inside the body of a patient into the first portion 110 of the separation device 100. The purified target liquid passage 12 is configured to guide the purified target liquid F12 out from the first portion 110 of the separation device 100 and to introduce the purified target liquid F12 into the body of the patient. The separation device 100 is constituted of, for example, a dialyzer. The first portion 110 is an internal space of hollow fibers constituted of a dialysis membrane. As will be described later, the second portion 120 is a space around the hollow fibers through which a dialysate and/or a regenerated liquid flows.

In the present embodiment, the separation device 100 is configured as a "counter flow-type separation device" in which a flow direction of the unpurified target liquid F1 in the first portion 110 and a flow direction of a regenerated liquid F22 in the second portion 120 oppose each other or are opposite orientations. On the other hand, in another embodiment, the separation device 100 may be configured as a "parallel flow-type separation device" in which the flow direction of the unpurified target liquid F11 in the first portion 110 and the flow direction of the regenerated liquid F22 in the second portion 120 are parallel to each other or are the same orientation.

The filtration dialysate passage F21 guides the filtration dialysate F21 out from the second portion 120 of the separation device 100. The effluent passage 24 discards the filtration dialysate F21 introduced from the filtration dialysate passage 21 as an effluent F41.

The dialysate passage 412 is configured to directly introduce the dialysate F41 into an introducing portion provided upstream of a guiding portion of the second portion 120. In this case, for example, the "dialysate" means a fresh dialysate.

The first flow rate adjustment device 36 is, for example, a flow control valve, a pump, a mass flow controller, or the like and is provided in at least one of the filtration dialysate passage 21 and the effluent passage 24. The first flow rate adjustment device 36 adjusts a flow rate of the filtration dialysate F21. In particular, the first flow rate adjustment device 36 is provided so as to control the flow rate of the filtration dialysate F21 to or below the flow rate of the unpurified target liquid F11.

The concentration sensor 35 is a sensor that outputs a signal according to a concentration of the target substance in the target liquid and is provided in at least one of the filtration dialysate passage 21 and the effluent passage 24. At this point, concentration sensors 35 of different types may be provided so as to enable respective concentrations of a plurality of target substances to be measured. Providing the concentration sensor 35 within a passage through which the filtration dialysate F21 passes enables the concentration of the target substance in the unpurified target liquid F11 to be estimated without having to provide the concentration sensor 35 inside the human body. In addition, as will be described later in a result, the concentration sensor 35 measures the concentration of the target substance while the first flow rate adjustment device 36 controls the flow rate of the filtration dialysate F21 to or below a flow rate of the unpurified target liquid F11. Therefore, the concentration of the target substance present in the filtration dialysate F21 or the effluent F211 can be referenced as the concentration of the target substance in the unpurified target liquid F11. Since a composition of the unpurified target liquid can be determined based on a measurement result from the concentration sensor 35, the target liquid purification device can adjust flow rates of the respective liquids in the target liquid purification device according to the concentration of the target substance. In addition, a degree of purification can be quantified and target liquid purification can be ended at an appropriate timing. For these reasons, the target liquid can be purified without having to add more dialysate or replenishment liquid than necessary.

While the concentration sensor 35 is preferably provided on a downstream of the first flow rate adjustment device 36, the concentration sensor 35 may also be provided on an upstream side of the first flow rate adjustment device 36. In addition, both the concentration sensor 35 and the first flow rate adjustment device 36 may be provided in one of the filtration dialysate passage 21 and the effluent passage 34 or may be provided at different locations, respectively.

Typically, during target liquid purification, since a flow of the dialysate side tends to be uneven, the flow rate of the dialysate F412 is set to approximately twice that of the unpurified target liquid F11. On the other hand, when the concentration measurement device 2 measures the concentration of the target substance, the flow rate of the dialysate F412 is set the same as the flow rate of the unpurified target liquid F11 or lower than the flow rate of the unpurified target liquid F11. In this case, since the unpurified target liquid F11 comes into prolonged contact with the porous membrane 102, the concentration of the unpurified target liquid F11 is approximately equal to the concentration of the filtration dialysate F21. In addition, since the amount of water that can be removed from the unpurified target liquid F11 is proportional to the increase in the flow rate of the filtration dialysate F21 compared to the replenishment liquid F41, a flow rate of the ultrafiltration liquid F0 can also be controlled.

In addition, in the present embodiment, the flow rate of the dialysate F412 is approximately equal to a sum of the flow rate of the filtration dialysate F21 and the flow rate of the ultrafiltration liquid F0. Since the filtration dialysate F21 and the regenerated liquid F22 flow within a flow path with a constant volume, when the flow rate of the dialysate F412 and a sum of the flow rate of the filtration dialysate F21 and the flow rate of the ultrafiltration liquid F0 are approximately the same, the unpurified target liquid F11 is equal to the sum of the flow rate of the purified target liquid F12 and the flow rate of the ultrafiltration liquid F0. In other words, the flow rate of the ultrafiltration liquid F0 is equal to a difference between the flow rates of the dialysate F41 and the filtration dialysate F21.

The target liquid purification device according to the present embodiment comprises the concentration measurement device 2, the control device 3, and the notification device 4 as shown in FIG. 1 in addition to the main body portion 1 described above. The concentration measurement device 2 and the control device 3 include: a storage device (a memory such as a RAM, a ROM, or an EEPROM, an SSD, an HDD, or the like) that stores and retains programs (software) and data; an arithmetic processing unit (a single-core processor, a multi-core processor, a CPU, or the like) that reads necessary programs and/or data from the storage device and performs predetermined arithmetic processing; an I/O circuit, and the like. As will be described later, the concentration measurement device 2 and the control device 3 execute control of the target liquid purification device as a whole by measuring the concentration of the target substance based on a signal output by the concentration sensor 35 while controlling flow rates adjusted by the first flow rate adjustment device 36. The control device 3 is provided with an abnormality detection device 301, and the abnormality detection device 301 detects an abnormality according to a concentration of the target substance measured by the measurement device as will be described later.

The notification device 4 comprises an output interface 402. The notification device 4 is an audio output device, an image output device, a personal computer, a mobile phone (smartphone), or the like, and includes any information terminal device. For example, the notification device 4 is configured to notify an abnormality obtained by the abnormality detection device 301 and to present various pieces of information during target liquid purification.

(Second embodiment)

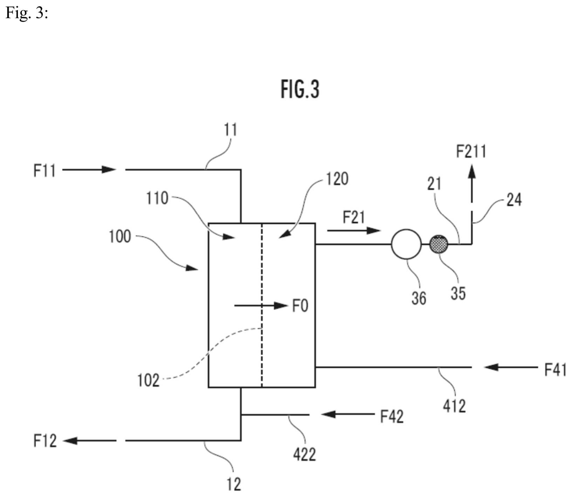

FIG. 3 shows a schematic diagram related to a second embodiment of the main body portion 1 in FIG. 1. The second embodiment relates to a target liquid purification device according to the first embodiment, further comprising a replenishment liquid passage 422 for introducing a replenishment liquid F42 into the main body portion 1. In this case, the replenishment liquid passage 422 is configured to introduce the replenishment liquid F422 into at least one of the unpurified target liquid passage 11, the purified target liquid passage 12, and the dialysate passage 412. Since other components are substantially similar to those of the first embodiment, descriptions of overlapping parts will not be repeated.

The replenishment liquid passage 422 may be provided in any one of the passages described above or may be simultaneously provided in a plurality of passages. For example, the replenishment liquid passage 422 may be configured to introduce the replenishment liquid F422 into both the unpurified target liquid passage 11 and the dialysate passage 412. In addition, the replenishment liquid passage 422 is preferably configured to introduce the replenishment liquid F422 on a downstream side of a position where the concentration sensor 35 is provided. In this case, since the unpurified target liquid F11 comes into prolonged contact with the porous membrane 102 when the concentration measurement device 2 measures the concentration of the target substance, the concentration of the target substance in the filtration dialysate F21 can be accurately measured. Furthermore, the replenishment liquid passage 422 is not limited to the embodiment described above and may be configured to introduce the replenishment liquid F422 on a upstream side of the position where the concentration sensor 35 is provided. In this case, the flow rate of the replenishment liquid is adjusted by a third flow rate adjustment device 32 to be described later. In addition, by adjusting the flow rate of the replenishment liquid when the concentration measurement device 2 measures the concentration based on a signal from the concentration sensor 35, the unpurified target liquid F11 can be brought into prolonged contact with the porous membrane 102. Furthermore, by recognizing the flow rate of the filtration dialysate F21 and the flow rate of the replenishment liquid F422 and multiplying an appropriate correction coefficient based on the flow rate ratio, the concentration of the target substance in the unpurified target liquid F422 can be accurately measured.

Here, the main body portion 1 may comprise a second flow rate adjustment device 31 which adjusts a flow rate of the first liquid F211 that is discharged from the target liquid purification device and the third flow rate adjustment device 32 which adjusts a flow rate of the replenishment liquid F42 that is introduced into the replenishment liquid passage 422. The second flow rate adjustment device 31 is, for example, a flow control valve or a mass flow controller and is provided in the effluent passage 24. The third flow rate adjustment device 32 is, for example, a flow control valve or a mass flow controller and is provided in the replenishment liquid passage 422 or the dialysate passage 412.

As will be described later, the second flow rate adjustment device 31 and the third flow rate adjustment device 32 may be controlled by the control device 3 so as to adjust respective flow rates according to a concentration of the target substance measured by the concentration sensor 35. In doing so, the control device 3 may control the second flow rate adjustment device 31 and the third flow rate adjustment device 32 so that each adjusts the flow rate so as to reduce a deviation between an introduction amount of the replenishment liquid F41 and a discharge amount of the first liquid F211 or to reduce a deviation between a total amount of the introduction amount of the replenishment liquid F41 and an amount of ultrafiltration and the discharge amount of the first liquid F212. When control is exercised in this manner, since the unpurified target liquid F11 comes into prolonged contact with the porous membrane 102, the concentration of the target substance in the unpurified target liquid F11 is approximately equal to the concentration of the target substance in the filtration dialysate F21. In addition, since the amount of water that can be removed from the unpurified target liquid F11 is proportional to the increase in the flow rate of the filtration dialysate F21 compared to the replenishment liquid F41, a flow rate of the ultrafiltration liquid F0 can also be controlled.

(Third embodiment)

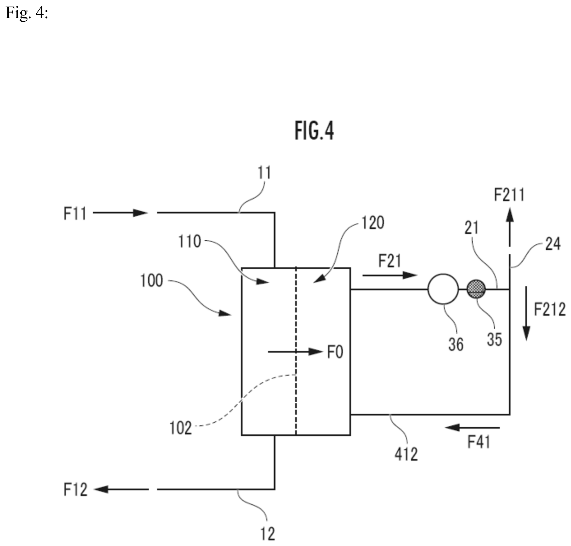

FIG. 4 shows a schematic diagram related to a third embodiment of the main body portion 1 in FIG. 1. The third embodiment relates to a target liquid purification device according to the first embodiment, further comprising a component having a branching portion at a downstream end portion of the filtration dialysate passage 21 that branches the filtration dialysate F21 into the first liquid F211 and the second liquid F212. Here, the effluent passage 24 branches from the filtration dialysate passage 21 and is configured to discard the first liquid F211 being a part of the filtration dialysate F21 as an effluent. In addition, the filtration dialysate passage 21 is configured to introduce the second liquid F212 being a remainder of the filtration dialysate F21 as a dialysate F41 into an introducing portion of the dialysate passage 412. In other words, the third embodiment relates to a circulating-type target liquid purification device.

In addition, the main body portion 1 of the third embodiment may further comprise the replenishment liquid passage 422 for introducing the replenishment liquid F42 into the main body portion 1 in a similar manner to the second embodiment. In this case, the replenishment liquid passage 422 is configured to introduce the replenishment liquid F422 into at least one of the unpurified target liquid passage 11, the purified target liquid passage 12, and the dialysate passage 412. Since other components are substantially similar to those of the first or second embodiment, descriptions of overlapping parts will not be repeated.

(Fourth embodiment)

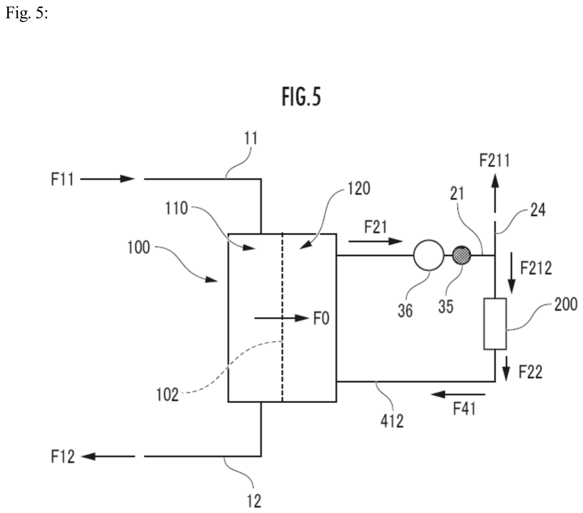

FIG. 5 shows a schematic diagram related to a fourth embodiment of the main body portion 1 in FIG. 1. The fourth embodiment relates to a target liquid purification device according to the third embodiment, further comprising a regenerating unit 200 provided between a branching point of the effluent passage 24 and an introducing portion of the dialysate passage 412 in the filtration dialysate passage 21. The regenerating unit 200 includes an adsorbent that adsorbs the target substance. By bringing the second liquid F212 introduced from the filtration dialysate passage 21 into contact with the adsorbent, the regenerating unit 200 removes at least one target substance from the second liquid F212 and generates the regenerated liquid F22 with a reduced concentration of the target substance. In addition, the regenerating unit 200 is configured to introduce the regenerated liquid F22 into the introducing portion of the dialysate passage 412 as the dialysate F41. Since other components are substantially similar to those of the third embodiment, descriptions of overlapping parts will not be repeated.

(Configuration of regenerating unit)

The regenerating unit 200 includes an adsorbent that adsorbs the target substance. As the adsorbent, activated carbon and/or adsorbents based on activated carbon, porous adsorbents, cation exchange resins, anion exchange resins, zirconia-based ceramics, adsorbents constituted of zeolites, and mixtures thereof may be adopted. The regenerating unit 200 is configured to remove at least a part of the target substance and to generate the regenerated liquid F22 with a reduced concentration of the target substance by bringing a part of the filtration dialysate (second liquid F212) into contact with the adsorbent.

In the fourth embodiment, the second liquid F212 is configured to be introduced into the regenerating unit 200. The effluent passage 24 branches from the filtration dialysate passage 21 and is configured to discard, among a first liquid F211 and the second liquid F212 as a result of the filtration dialysate F21 being separated, the first liquid F211 as an effluent. A flow rate of the effluent in the effluent passage 24 (discarded amount of filtration dialysate F21) may be appropriately controlled based on an inflow rate of a replenishment liquid (fresh dialysate) into the regenerated liquid passage 22 and/or an outflow rate of the regenerated liquid F22 from the regenerated liquid passage 22, taking into consideration the flow rate of the regenerated liquid F22 in the second portion 120 of the separation device 100. The regenerated liquid passage 22 is configured to introduce the regenerated liquid F22 from the regenerating unit 200 into the second portion 120 of the separation device 100.

In the present embodiment, the regenerating unit 200 is preferably constituted of at least one or more regenerating circuits. While respective regenerating circuits are preferably connected in series when there are two or more regenerating circuits, the regenerating circuits are not limited thereto and may be connected in parallel.

While the second to fourth embodiments have been described as modifications of the first embodiment, configurations of the respective embodiments may be combined. For example, the configuration of the second embodiment may be combined with the configuration of the fourth embodiment.

(Concentration measurement method)

Next, a measurement method of a concentration of a target substance in a target liquid in a target liquid purification method that is executed by the target liquid purification device common to the first to fourth embodiments will be described.

The concentration of the target substance is measured by, for example, healthcare professionals or patients themselves to determine whether the target liquid has been sufficiently purified upon receiving a request signal transmitted via an input interface of the target liquid purification device. In addition, for example, the concentration of the target substance is automatically measured at the start of target liquid purification such as when a degree of opening of the fourth flow rate adjustment device 2013 is adjusted according to the concentration of the target substance or when a designated time has elapsed from a previous concentration measurement as in the fourth embodiment.

When measuring the concentration of the target substance, the output of the first flow rate adjustment device 36 becomes an output of the first flow rate adjustment device 36 for setting the filtration dialysate F21 the same as the flow rate of the unpurified target liquid F11 or lower than the flow rate of the unpurified target liquid F11. Accordingly, since the unpurified target liquid F11 does not come into contact with the porous membrane 102 more than necessary, the concentration of the unpurified target liquid F11 and the concentration of the filtration dialysate F21 are substantially equal. In addition, since the amount of water that can be removed from the unpurified target liquid F11 is proportional to the increase in the flow rate of the filtration dialysate F21 compared to the replenishment liquid F41, a flow rate of the ultrafiltration liquid $F_0$ can also be controlled.

When the first flow rate adjustment device 36 sets the filtration dialysate F21 the same as the flow rate of the unpurified target liquid F11 or lower than the flow rate of the unpurified target liquid F11, the concentration measurement device 2 measures the concentration of the target substance in a target liquid based on the output signal of the concentration sensor 35.

Note that, here, a value of the concentration of the target substance may be recorded or output to the notification device simultaneously with the measurement. Accordingly, healthcare professionals and patients can readily comprehend the concentration of the target substance during target liquid purification and quantitatively determine a degree of purification of the target liquid.

Additionally, when the replenishment liquid passage 422 is configured to introduce the replenishment liquid F422 on a downstream side of the position where the concentration sensor 35 is provided, the concentration measurement device 2 may be configured to accurately measure the concentration of the target substance in the unpurified target liquid F422 by multiplying the concentration of the target substance by an appropriate correction coefficient based on a flow rate ratio between the flow rate of the filtration dialysate F21 and the flow rate of the replenishment liquid F422.

(Abnormality detection method)

In addition to components of the embodiments described above, the target liquid purification device comprises: the abnormality detection device 301 configured to detect an abnormality based on a time-series variation in a concentration of a target substance; and the notification device 4 configured to notify an abnormality detected by the abnormality detection device 301 and the like.

The abnormality detection device 301 is a device which constitutes a part of the control device 3 and which detects an abnormality based on a time series of the concentration of the target substance. In this case, a time-series variation in the concentration of the target substance refers to, for example, information representing the concentration measured by the concentration measurement device 2 described above for each time series. In this case, values of a plurality of target substances may be adopted as values of the concentration. Based on time-series information of the concentrations of the plurality of target substances, an abnormal value is calculated statistically or using a method of machine learning. For example, when the value of the concentration does not vary, it can be determined that dialysis is not performed sufficiently. In addition, when a specific substance is present in a larger amount than other substances, it can be determined that the composition of the replenishment liquid and/or the dialysate is insufficient.

The notification device 4 is connected to the target liquid purification device via wireless communication such as a network or via wired communication such as a cable. The notification device 4 is, for example, software installed on a personal computer, a smartphone, or the like. The notification device 4 is carried by, for example, a patient using the target liquid purification device, their family members, their assigned healthcare professionals, or the like.

When the abnormality detection device 301 detects an abnormality, the detection of the abnormality is notified to the output interface 402 of the notification device 4. Accordingly, a user can recognize deterioration of the target liquid purification device and/or target liquid purification processes and the like and, based on the recognition, reliably execute purification of the target liquid. Furthermore, since healthcare professionals can recognize the presence or absence of disease, disease risk factors, or the like in advance, the healthcare professionals can effectively perform target liquid purification in order to maintain the health of patients using the target liquid purification device.

Example

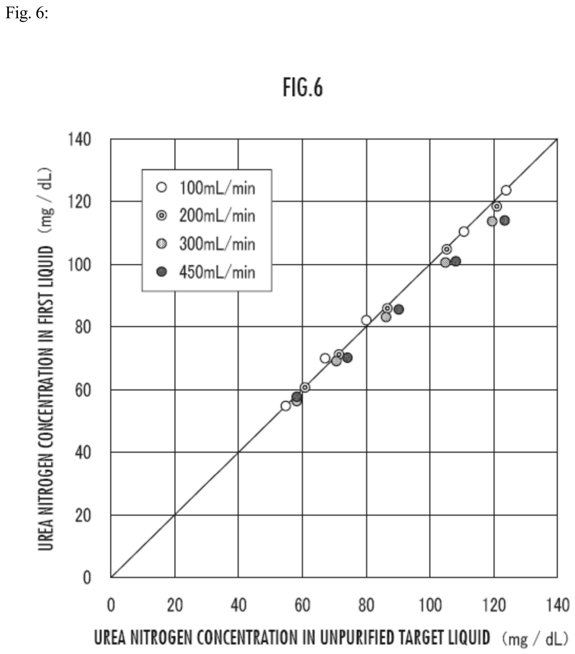

A first example is a target liquid purification device with the configuration of the first embodiment. FIG. 6 is a diagram showing a concentration of urea nitrogen as a target substance in the unpurified target liquid F11 side and a concentration of urea nitrogen in the first liquid F211 (same as filtration dialysate F21) when the flow rate of the dialysate F41 is set to 100, 200, 300, and 450 mL/min in the target liquid purification device according to the first example. Note that, here, the unpurified target liquid F11 (blood flow rate) is 250 mL/min. Referring to FIG. 6, when the flow rate of the dialysate F41 was greater than the flow rate of the unpurified target liquid F11 (replenishment liquid F41 flow rate: 100, 200 mL/min), the concentration of urea nitrogen in the unpurified target liquid F11 was approximately the same as the concentration of urea nitrogen in the first liquid F211. On the other hand, when the flow rate of the dialysate F41 was greater than the flow rate of the unpurified target liquid F11 (dialysate F41 flow rate: 300, 450 mL/min), the concentration of urea nitrogen in the first liquid F211 was lower than the concentration of urea nitrogen in the unpurified target liquid F11.

Here, when a removal performance achieved after a single pass through the target liquid purification device is expressed by a clearance CL index, the clearance CL is expressed by the following equation (1), using a urea nitrogen concentration $C_{BI}$ in the unpurified target liquid F11, a urea nitrogen concentration $C_{BO}$ in the purified target liquid F12, and a flow rate $Q_B$ of the unpurified target liquid F11.

$$CL = ((C_{BI} - C_{BO})/C_{BI}) * Q_B \ldots (1)$$

Here, CL being 230 to 250 mL/min when $Q_B$ is 250 mL/min means that approximately 100% of urea nitrogen is removed in a single pass of the target liquid purification device. In addition, based on material balance, a concentration of urea nitrogen in the first liquid F211 at this time is expressed by the following equation (2), using a concentration of urea nitrogen in the dialysate F41, a concentration of urea nitrogen in the first liquid F211, and a flow rate of the dialysate F41 as $C_{DI}$, $C_{DO}$, and $Q_D$, respectively.

$$Q_B*(C_{BI} - C_{BO}) = Q_D*(C_{DO} - C_{DI}) \ldots (2)$$

Furthermore, using an overall mass transfer area coefficient KoA yields the following relational expression (3).

$$(C_{BI} - C_{BO})/(C_{BI} - C_{DI}) = (1 - \exp(KoA(1/Q_{BI} - 1/Q_{BO})))/(Q_{BI}/Q_{DI} - \exp(KoA(1/Q_{BI} - 1/Q_{BO}))) \tag{3}$$

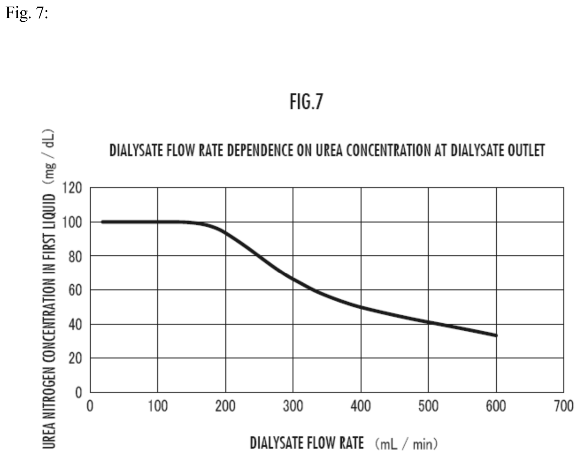

$C_{DO}$ can be determined based on the above relational expressions (1) to (3). At this point, when operating conditions are set to $Q_B = 200$ mL/min and $C_{BI} = 100$ mg/dL, KoA can be considered to be approximately 3000 in the case of urea nitrogen. When a length of the separation device (dialyzer) is set to 25 cm and a membrane area to 1.5 $m^2$, $C_{DO}$ when $Q_D$ is varied is calculated, yielding FIG. 7.

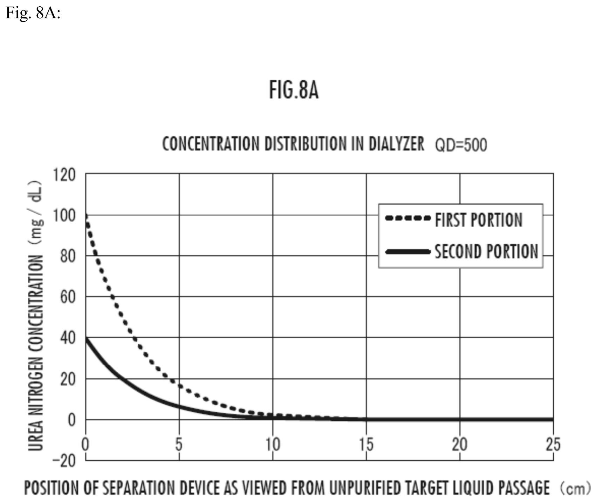

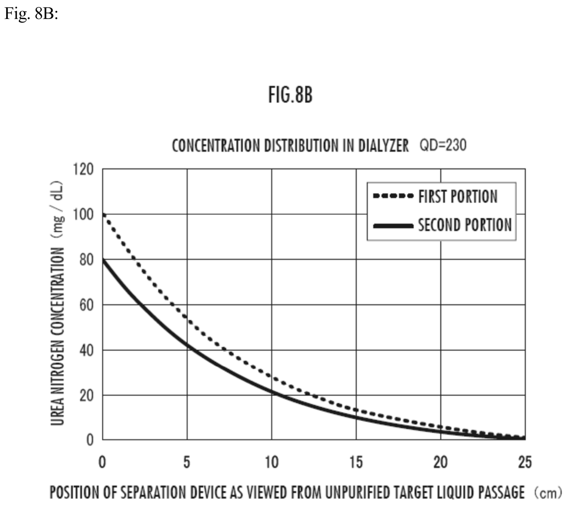

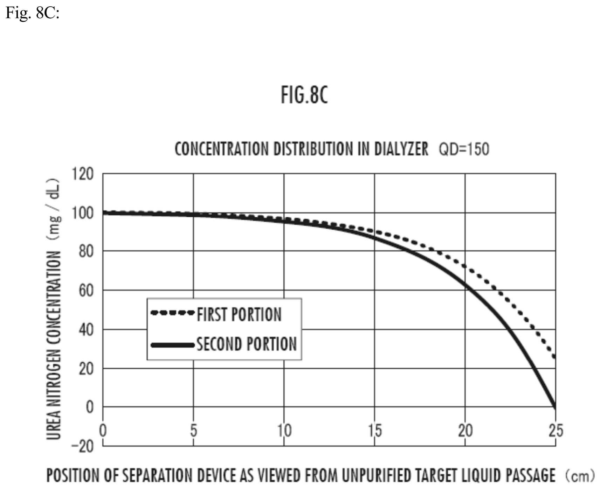

Referring to FIG. 7, when the flow rate of the dialysate F41 becomes smaller than approximately 200 mL/min, which is approximately equal to the flow rate of the unpurified target liquid F11, the first liquid F211 has a concentration of approximately 100 mg/dL, which is approximately equal to that of the unpurified target liquid F11. This is attributable to an extremely high transfer rate of urea nitrogen from the unpurified target liquid F11 to the separation device in the target liquid purification device. At this point, the concentration distribution of urea nitrogen in the unpurified target liquid F11 side and in the dialysate F41 side within the target liquid purification device can be calculated as shown in FIG. 8.

In FIG. 8, an axis of abscissa represents a position in a length direction of the separation device 100 (where an inflow portion of the unpurified target liquid F11 into the separation device is 0 and an outflow portion to the purified target liquid F12 is 25 on the axis of abscissa), and an axis of ordinate represents urea nitrogen concentration. Here, solid lines represent the urea nitrogen concentration in the first portion 110 and dotted lines represent the urea nitrogen concentration in the second portion 120. Referring to FIG. 8A, when the flow rate of the replenishment liquid F41 is 500 mL/min, conceivably, approximately 100% of the urea nitrogen in the unpurified target liquid F11 is being removed (a value of urea nitrogen at 25 cm is approximately zero). Referring to FIG. 8B, when the flow rate of the dialysate F41 is 230 mL/min, approximately 100% of the urea nitrogen is similarly being removed. At this point, a y-intercept value representing the urea nitrogen concentration of the first liquid F211 is 40 mg/dL when the flow rate of the dialysate F41 is 500 mL/min, which is lower than the urea nitrogen concentration of the unpurified target liquid F11. On the other hand, as the flow rate of the dialysate F41 decreases, the urea nitrogen concentration of the first liquid F211 increases. Referring to FIG. 8C, in the graph showing a flow rate of 150 mL/min for the dialysate F41, the urea nitrogen concentration in the unpurified target liquid F11 and the urea nitrogen concentration in the first liquid F211 are each approximately 100 mg/dL and can be considered equivalent. Accordingly, even without having to provide the concentration sensor 35 in the unpurified target liquid F11 (blood), the urea nitrogen concentration in the unpurified target liquid F11 can be determined by measuring the concentration of urea nitrogen in either the first liquid F211 or the filtration dialysate F21. Accordingly, since the processing described earlier is executed, a total amount of the dialysate F41 in the target liquid purification device can be reduced and, at the same time, purification can be reliably executed.

While the results have been presented using values of urea nitrogen in the example, similar results were obtained with substances other than urea nitrogen that exhibit high permeability through the separation membrane. Although not specifically illustrated, similar results were obtained using potassium ions. Therefore, such a target liquid purification device can accurately measure the concentrations of a plurality of target substances, enabling overall control of the target liquid purification device based on the concentrations.

Based on the above, the target liquid purification device enables control that reliably processes a target substance from a target liquid while reducing the amount of dialysate. Therefore, the target liquid purification device can be used in home dialysis and the like. In addition, if an abnormality occurs during home dialysis, since the abnormality can be transmitted to remote medical professionals and the like, reliable dialysis treatment can be provided. In addition, a target liquid purification device can be provided which is capable of reliably performing dialysis and accurately measuring a concentration of a target substance in blood while reducing a total volume of a dialysate used in the dialysis despite having a structure which is simple and safe enough to have no adverse effect on the human body and which does not require drawing blood.

It should be noted that the present invention is not limited to the embodiments or the examples described above, and the scope of the present invention can clearly be modified or altered within the scope obvious to those skilled in the art. Therefore, it should be obvious that the scope of the present invention is not limited to the embodiments or the examples described above but also includes modifications and alterations thereof.

Reference Signs List

| | |
|---|---|
| 1 | main body portion |
| 2 | concentration measurement device |
| 3 | control device |
| 4 | notification device |
| 11 | unpurified target liquid passage |
| 12 | purified target liquid passage |
| 21 | filtration dialysate passage |
| 22 | regenerated liquid passage |
| 24 | effluent passage |
| 31 | second flow rate adjustment device |
| 32 | third flow rate adjustment device |
| 35 | concentration sensor |

| 36 | first flow rate adjustment device |
|---|---|
| 100 | separation device |
| 102 | porous membrane |
| 110 | first portion |
| 120 | second portion |
| 200 | regenerating unit |
| 201 | regenerating circuit |
| 202 | regenerating circuit |
| 221 | regenerated liquid branch passage |
| 222 | regenerated liquid branch passage |
| 301 | abnormality detection device |
| 401 | input interface |
| 402 | output interface |
| 412 | dialysate passage |
| 422 | replenishment liquid passage |
| 2011 | first circuit (regenerating circuit 201) |
| 2012 | second circuit (regenerating circuit 201) |
| 2013 | fourth flow rate adjustment device (regenerating circuit 201) |
| 2021 | first circuit (regenerating circuit 202) |
| 2022 | second circuit (regenerating circuit 202) |
| 2023 | fourth flow rate adjustment device (regenerating circuit 202) |
| F11 | unpurified target liquid |
| F12 | purified target liquid |
| F21 | filtration dialysate |
| F211 | first liquid (effluent) |
| F212 | second liquid |
| F22 | regenerated liquid |
| F41 | dialysate |
| F42 | replenishment liquid. |

**Claims**

1. A target liquid purification device that removes at least one of a plurality of target substances from an unpurified target liquid, the target liquid purification device comprising:

a separation device including a porous membrane having a pore size through which the target substances contained in the unpurified target liquid can pass and a first portion and a second portion separated by the porous membrane, the separation device being configured to generate a filtration dialysate by introducing the unpurified target liquid containing the target substances from the first portion into the second portion while passing the unpurified target liquid through the porous membrane and to generate a purified target liquid by removing the target substances contained in the unpurified target liquid by the pass-through;
an unpurified target liquid passage configured to introduce the unpurified target liquid into the first portion of the separation device;
a purified target liquid passage configured to guide the purified target liquid out from the first portion of the separation device;
a filtration dialysate passage configured to guide the filtration dialysate out from a guiding portion of the second portion of the separation device;
an effluent passage configured to discard the filtration dialysate from the filtration dialysate passage as an effluent;
a dialysate passage configured to introduce a dialysate into an introducing portion provided upstream of the guiding portion of the second portion;
a concentration sensor which is provided in at least one of the filtration dialysate passage and the effluent passage and which is configured to output a signal according to the concentration of the target substances in the filtration dialysate;
a flow rate adjustment device which is provided in at least one of the filtration dialysate passage and the effluent passage and which is configured to adjust a flow rate of the filtration dialysate; and
a concentration measurement device configured to measure the concentration of the target substances based on the output signal of the concentration sensor in a time period where the flow rate adjustment device controls the flow rate of the filtration dialysate to or below a flow rate of the unpurified target liquid.

2. The target liquid purification device according to claim 1, further comprising

a replenishment liquid passage configured to introduce a replenishment liquid into the target liquid purification device, wherein
the replenishment liquid passage is configured to introduce the replenishment liquid into at least one of the unpurified target liquid passage, the purified target liquid passage, and the dialysate passage.

3. The target liquid purification device according to claim 1, wherein

the effluent passage branches from the filtration dialysate passage and is configured to discard a first liquid being a part of the filtration dialysate as an effluent; and
the filtration dialysate passage is configured to introduce a second liquid being a remainder of the filtration dialysate as a dialysate into an introducing portion of the dialysate passage.

4. The target liquid purification device according to claim 3, further comprising

a replenishment liquid passage configured to introduce a replenishment liquid into the target liquid purification device, wherein
the replenishment liquid passage is configured to introduce the replenishment liquid into at least one of the unpurified target liquid passage, the purified target liquid passage, the filtration dialysate passage, and the dialysate passage.

5. The target liquid purification device according to claim 3, comprising

a regenerating unit which is provided between a branching point of the effluent passage and the introducing portion of the dialysate passage in the filtration dialysate passage, which includes an adsorbent configured to adsorb the target substances, and which is configured to remove at least one of the target substances from the second liquid by bringing the second liquid having been introduced from the filtration dialysate passage in contact with the adsorbent and to generate a regenerated liquid with a reduced concentration of the target substances, wherein
the regenerating unit is configured to introduce the regenerated liquid as a dialysate into the introducing portion of the dialysate passage.

6. The target liquid purification device according to claim 5, further comprising

a replenishment liquid passage configured to introduce a replenishment liquid into the target liquid purification device, wherein
the replenishment liquid passage is configured to introduce the replenishment liquid into at least one of the unpurified target liquid passage, the purified target liquid passage, the filtration dialysate passage, and the dialysate passage.

7. The target liquid purification device according to claim 6, comprising
a effluent liquid flow rate adjustment device configured to adjust a flow rate of the first liquid and a replenishment liquid flow rate adjustment device configured to adjust a flow rate of the replenishment liquid.

8. The target liquid purification device according to any one of claims 1 to 7, comprising:

an abnormality detection device configured to detect an abnormality based on a time-series variation in the concentration of the target substances; and
a notification device configured to notify an abnormality detected by the abnormality detection device.

Fig. 1:

## FIG.1

TARGET LIQUID PURIFICATION DEVICE

| NOTIFICATION DEVICE | MAIN BODY PORTION | CONCENTRATION MEASUREMENT DEVICE |

4

1

2

CONTROL DEVICE

3

Fig. 2:

## FIG.2

11

F11 →

F211 ↑

120

21

24

110

F21 →

100

F0 →

36

35

102

F41 ←

412

F42

F12 ←

12

Fig. 3:

FIG.3

Fig. 4:

FIG.4

Fig. 5:

FIG.5

Fig. 6:

Fig. 7:

FIG.7

DIALYSATE FLOW RATE DEPENDENCE ON UREA CONCENTRATION AT DIALYSATE OUTLET

Fig. 8A:

FIG.8A

CONCENTRATION DISTRIBUTION IN DIALYZER  QD=500

Fig. 8B:

FIG.8B

Fig. 8C:

**FIG.8C**

## INTERNATIONAL SEARCH REPORT

| International application No. |
| --- |
| **PCT/JP2025/001903** |

**A. CLASSIFICATION OF SUBJECT MATTER**

*A61M 1/34*(2006.01)i; *A61M 1/16*(2006.01)i
FI:    A61M1/34 120; A61M1/16 117; A61M1/16 190; A61M1/16 111

According to International Patent Classification (IPC) or to both national classification and IPC

**B. FIELDS SEARCHED**

Minimum documentation searched (classification system followed by classification symbols)

A61M1/00-1/38

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2016-47126 A (ASAHI KASEI MEDICAL CO., LTD.) 07 April 2016 (2016-04-07) entire text, all drawings | 1-8 |
| A | JP 11-76395 A (SHIBUYA CORP.) 23 March 1999 (1999-03-23) entire text, all drawings | 1-8 |
| A | JP 10-71201 A (TERUMO KABUSHIKI KAISHA) 17 March 1998 (1998-03-17) entire text, all drawings | 1-8 |

☐ Further documents are listed in the continuation of Box C.    ☑ See patent family annex.

| | |
| --- | --- |
| *     Special categories of cited documents: | "T"    later document published after the international filing date or priority date and not in conflict with the application but cited to understand the principle or theory underlying the invention |
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
| --- | --- |
| **07 March 2025** | **25 March 2025** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

International application No.

**PCT/JP2025/001903**

| Patent document cited in search report | | | Publication date (day/month/year) | Patent family member(s) | Publication date (day/month/year) |
|---|---|---|---|---|---|
| JP | 2016-47126 | A | 07 April 2016 | (Family: none) | |
| JP | 11-76395 | A | 23 March 1999 | (Family: none) | |
| JP | 10-71201 | A | 17 March 1998 | (Family: none) | |

Form PCT/ISA/210 (patent family annex) (July 2022)

**REFERENCES CITED IN THE DESCRIPTION**

**Patent documents cited in the description**

- US 9302038 B